# EUROPÄISCHE PATENTANMELDUNG

(11) **EP 0 551 663 A1**
(43) Veröffentlichungstag der Anmeldung: **21.07.1993**
(21) Anmeldenummer: 92122179.2
(22) Anmeldetag: 31.12.1992
(51) Int. Cl.: C07D 211/90, A61K 31/44

(54) **2,6-Dimethyl-1-n-propyl-4-(4-trifluormethylphenyl)-1,4-Dihydropyridin-3,5-dicarbonsäureester, Verfahren zu seiner Herstellung und seine pharmazeutische Verwendung**

(30) Priorität: 14.01.1992 DE 4200714
(71) Anmelder: BAYER AG, 51368 Leverkusen (DE)
(72) Erfinder: Behner, Otto, Dr., W-5600 Wuppertal 1 (DE); Wollweber, Hartmund, Dr., W-5600 Wuppertal 1 (DE); Goldmann, Siegfried, Dr., W-5600 Wuppertal 11 (DE); Rosen, Bruno, Dr., W-5603 Wülfrath (DE); Zaiss, Siegfried, Dr., W-5600 Wuppertal 1 (DE)

(57) **Zusammenfassung**

Die Erfindung betrifft den neuen 2,6-Dimethyl-1-n-propyl-4-(4-trifluormethylphenyl)-1,4-dihydropyridin-3,5-dicarbonsäuredimethylester der allgemeinen Formel I 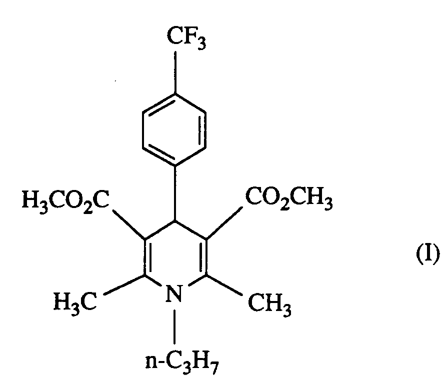
Verfahren zu seiner Herstellung und seine Verwendung als Arzneimittel bei ischämischen Erkrankungen, die mit Mikrozirkulationsstörungen verbunden sind. Diese Wirkung kann sowohl im peripheren als auch im cerebralen Gefäßsystem auftreten.

## Beschreibung

Die Erfindung betrifft den neuen 2,6-Dimethyl-1-n-propyl-4-(4-trifluormethylphenyl)-1,4-dihydropyridin-3,5-dicarbonsäuredimethylester, Verfahren zu seiner Herstellung und seine Verwendung als Arzneimittel bei ischämischen Erkrankungen, die mit Mikrozirkulationsstörungen verbunden sind. Diese Wirkung kann sowohl im peripheren als auch im cerebralem Gefäßsystem auftreten.

Es ist bereits bekannt, daß 1,4-Dihydropyridindicarbonsäureester eine calciumantagonistische oder calciumagonistische Wirkung besitzen, und somit als gefäß- und kreislaufbeeinflussende Mittel eingesetzt werden können [vgl. DOS 25 06 987; DOS 22 10 667].

In der EP 240 828 werden auch blutdrucksenkende 1,4-Dihydropyridine mit hämorheologischen Eigenschaften beschrieben.

Die vorliegende Erfindung betrifft den neuen 2,6-Dimethyl-1-n-propyl-4-(4-trifluormethylphenyl)-1,4-dihydropyridin-3,5-dicarbonsäuredimethylester der Formel (I), 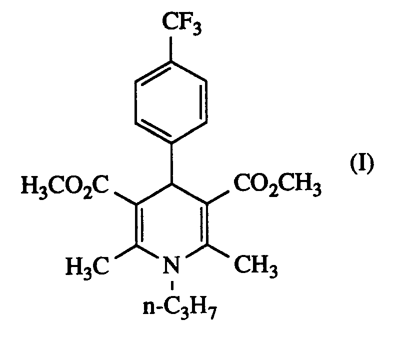
der überraschenderweise eine starke hämorheologische Wirkung besitzt und die Durchblutung, insbesondere die Mikrozirkulation verbessert und gleichzeitig blutdruckneutral ist. Er ist somit besonders geeignet zur Verwendung bei der Bekämpfung akuter und chronisch ischämischer Erkrankungen.

Die erfindungsgemäße Verbindung der Formel (I) kann nach üblichen Methoden hergestellt werden, z.B. indem man
[A] 2-(4-Trifluormethylbenzyliden)acetessigsäuremethylester der Formel (II) 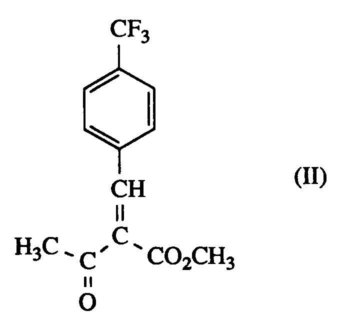 entweder direkt mit 3-n-Propylamino-crotonsäuremethylester, oder
   mit Acetessigsäuremethylester und n-Propylamin-Hydrochlorid
   in inerten Lösemitteln, gegebenenfalls in Anwesenheit einer Base / Säure umsetzt,
oder
[B] 4-Trifluormethylbenzaldehyd der Formel (III) 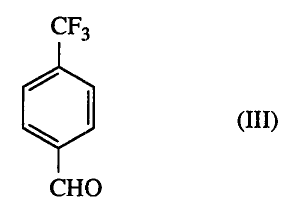 entweder mit Acetessigsäuremethylester und n-Propylaminhydrochlorid bzw. n-Propylamin und Pyridin-Hydrochlorid in Pyridin umsetzt,
oder
[C] zunächst unter Schutzgasatmosphäre Lewissäuren, vorzugsweise Titantetrachlorid, mit einer Base, vorzugsweise Piperidin, mit 3-n-Propylaminocrotonsäuremethylester in inerten Lösemitteln versetzt und anschließend 4-Trifluormethylbenzaldehyd der Formel (III) zufügt, oder
[D] 2,6-Dimethyl-4-(4-trifluormethylphenyl)-1,4-dihydropyridin-3,5-dicarbonsäuredimethylester der Formel (IV) 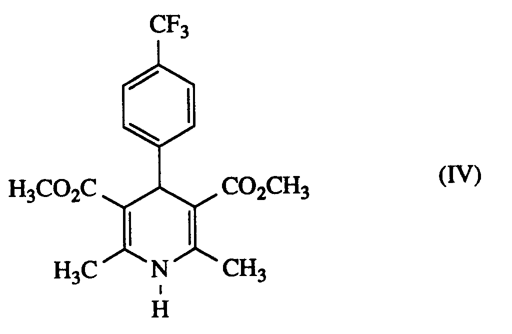 mit Alkylierungsmitteln, vorzugsweise n-Propyliodid oder Trifluormethansulfonsäure-n-propylester, in inerten Lösemitteln und in Anwesenheit einer Base umsetzt.

Das erfindungsgemäße Verfahren kann durch folgendes Formelschema beispielhaft erläutert werden: 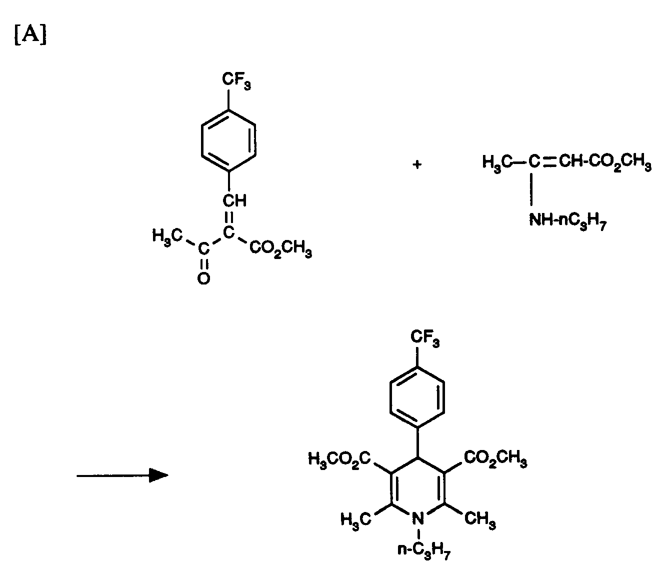 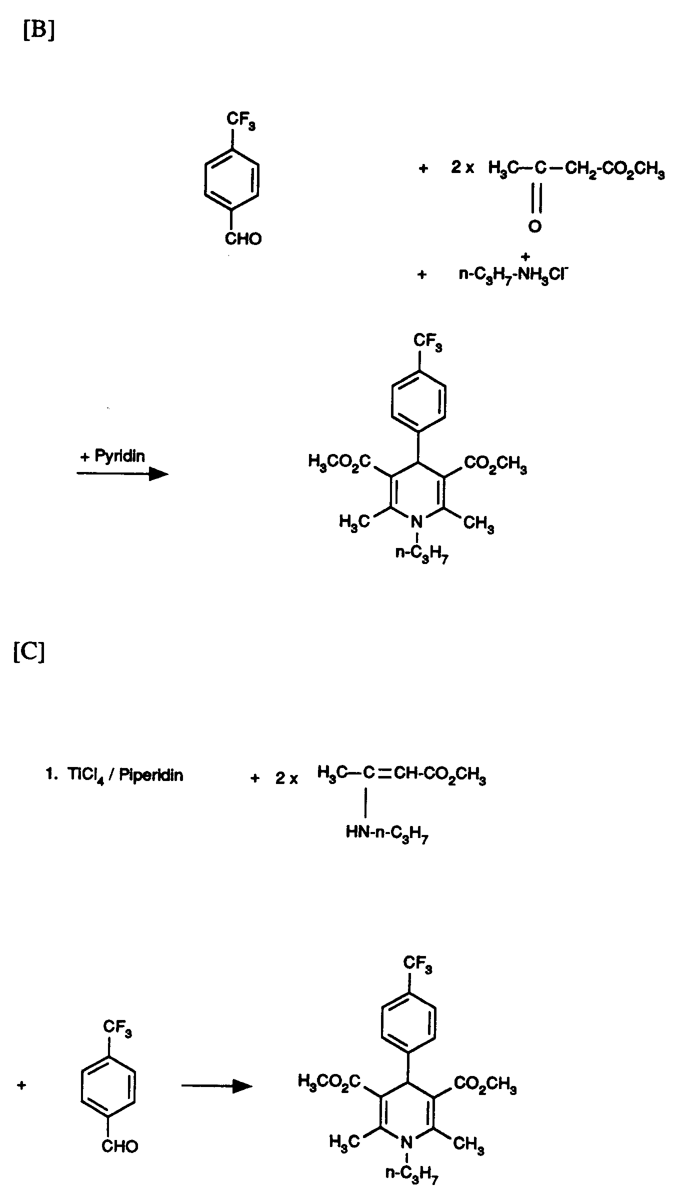 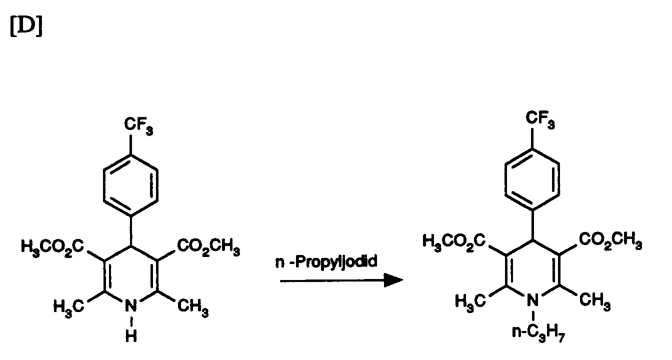
Als Lösemittel kommen Wasser oder organische Lösemittel in Frage, die sich unter den Reaktionsbedingungen nicht verändern. Hierzu gehören vorzugsweise Alkohole wie Methanol, Ethanol, Propanol, Isopropanol, Butanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind 1,2-Dimethoxyethan, Butanol und Pyridin.

Als Basen können in Abhängigkeit von den einzelnen Verfahrensschritten Hydride wie Natriumhydrid, Alkali- oder Erdalkalihydroxide, wie beispielsweise Natriumhydroxid oder Kaliumhydroxid, Alkoholate, wie Kalium-tert.butylat, oder Pyridin eingesetzt werden. Bevorzugt sind Natriumhydrid und Pyridin.

Als Säuren werden im allgemeinen Salzsäure oder Schwefelsäure eingesetzt.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A, B, C und D ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkriställisiert. In einigen Fällen kann es erforderlich sein, die erfindunsgemäßen Verbindungen durch Chromatographie zu reinigen.

Die Verbindung der Formel (II) ist bekannt und kann nach üblicher Methode hergestellt werden [vgl. H. Dornow und W. Sassenberg, Liebigs Ann. Chem. 602, 14 (1957)].

4-Trifluormethylbenzaldehyd (III) ist ebenso bekannt [vgl. Beilstein 7 (3), 1013].

Als Alkylierungsmittel bei dem Verfahren können beispielsweise n-Propylhalogenide, vorzugsweise n-Propyliodid, Trifluormethansulfonsäure-n-propylester oder Di-n-Propylsulfat eingesetzt werden.

Die Alkylierung wird in den oben aufgeführten Lösemitteln bei Temperaturen von 0°C bis +150°C, vorzugsweise bei Raumtemperaturen bis +100°C bei Normaldruck durchgeführt.

Die neue Verbindung zeigt ein nicht vorhersehbares, wertvolles pharmakologisches Wirkspektrum.

Verbunden mit einem gefäß- und blutdruckneutralem Verhalten in einem Dosis-Bereich bis mindestens 10 mg/kg i.v. und 30 mg/kg p.o. steigert sie die Durchblutung, insbesondere die Mikrozirkulation durch Beeinflussung der Verformbarkeit der Erythrozyten sowie der Inhibition der Aktivierung und Adhäsion der Leukozyten.

Die Blutdruckneutralität wird an folgenden, für Dihydropyridine typischen Modellen, ermittelt: In SH-Ratten nach p.o. Gabe durch Messung an der Schwanzarterie (Riva Rocci Methode) und an narkotisierten Wistar-Ratten nach i.v. Gabe. (Die Messung erfolgte über Katheter in A. carotis). Als blutdruckneutral wird diese Verbindung bezeichnet, da sie in beiden Testmodellen mit der angegebenen Dosis den Blutdruck nicht senkt. Der Abstand der therapeutischen Dosis und einer Blutdruckwirkung ist mindestens Faktor 100.

Die erfindungsgemäße Verbindung kann deshalb für die Herstellung von Arzneimitteln zur Behandlung von akuten und chronisch ischämischen Erkrankungen, wie Claudicatio intermittens, Myokardinfarkt, Gehirninfarkt sowie von Reperfusionsschäden und Schock eingesetzt werden.

Die folgenden in vitro und in vivo Tests zeigen die interessanten Wirkungen der speziell ausgewählten erfindungsgemäßen Verbindung.

### 1) Erythrozytenfunktion

Die Verformbarkeit von Erythrozyten spielt für die Entstehung und den Verlauf akuter oder chronisch ischämischer Erkrankungen eine wesentliche Rolle. Sie bestimmen die Viskosität des Bluts und damit seine Verteilung in der Mikrozirkulation. Die angewendeten Tests erfassen verschiedene Determinanten:
Test a) erfaßt die antihärmolytische Wirkung der Substanzen (ED₅₀, mol/l). Hierbei werden calciumbeladene Erythrozyten unter hohen Schubspannungen durch kleine Poren gepreßt, sodaß Hämoglobin als Ausdruck ihrer Hämolyse freigesetzt und gemessen wird. Die Verminderung der Hämoglobinfreisetzung ist die Meßgröße.
Test b) erfaßt die Viskosität von Erythrozytensuspensionen in Glaskapillaren (25 µm Durchmesser) bei niedrigen, in Gefäßgebieten hinter einer Stenose auftretenden Schubspannungen. Durch Erhöhung des extrazellulären Calciums steigt die Viskosität an.

### a) Antihämolytische Wirkung an Erythrozyten

Normale Erythrozyten werden unter hohen Schubspannungen hämolytisch. Besonders ausgeprägt ist die Hämolyse von calciumbeladenen Zellen. Dieses Maß für mechanische Stabilität wird zur Substanzcharakterisierung herangezogen. Meßgröße ist die Konzentration freien Hämoglobins im Medium.

### b) Viskosität in Glaskapillaren

Die biophysikalischen, für die Durchblutung relevanten Wechselwirkungen von Erythrozyten können in Glaskapillaren (Durchmesser 20-30 µm) untersucht werden. Die resultierende Viskosität hängt von dem Zustand der Zellen ab. Bei Calciumbeladung steigt die Viskosität an. Angegeben ist die prozentuale Verbesserung der Viskosität bezogen auf geschädigte, jedoch unbehandelte Kontrolle bei 0,7 Pa. Die Testdosis beträgt 10⁻⁸ g/ml.

**Tabelle I**

| Beispiel-Nr. | Effekt (%) |
|---|---|
| erfindungsgemäße Verbindung (I) | 190 |

### II) Leukozytenfunktion

Im Modell der Hamsterbackentasche kann die Mikrozirkulation direkt beobachtet werden. Meßgrößen sind die Leukozytenadhäsion sowie Gefäßdurchmesser und Erythrozytensenkungsgeschwindigkeit. Die Adhäsion wurde unter ischämischen und nicht ischämischen Versuchsbedingungen quantifiziert. Unter nicht ischämischen Bedingungen ist die Adhäsion im Bereich kleiner Venulen quantifiziert, unter ischämischen Bedingungen (10 min Durchblutungsstop) in kleinen Arteriolen. Die Ergebnisse der Kontrollversuche sind auf 100 % angeglichen. Als Testdosis werden jeweils 0,1 mg/kg i.v. gewählt, die Ergebnisse sind Abnahme in % der Kontrolle. Überraschenderweise zeigte sich, daß unter ischämischen Bedingungen die Substanz auch noch mit 0,03 mg/kg i.v. wirkte. Dies ist besonders günstig für die angestrebten Indikationen.

**Tabelle II**

| Beispiel-Nr. | nicht-ischämisch Kontrolle =100% | ischämisch Kontrolle =100% |
|---|---|---|
| erfindungsgemäße Verbindung (I) | 55% | 37% |

### III) Blutdruck

Der klinische Kenntnisstand zeigt, daß antiischämische Wirkungen von Dihydropyridinen häufig durch eine Vasodilatation überdeckt werden. Es war daher Ziel, blutdruckunwirksame (d.h. Abstand hämorheologische Wirkung und blutdrucksenkende Wirkung ≧ 10) DHP's zu finden. Die folgende Tabelle zeigt die Dosen, bei denen bei p.o.-Gabe (SH-Ratte) bzw. i.v.-Gabe (narkotisierte Wistarratte) eine Blutdrucksenkung eintritt.

**Tabelle III**

| Beispiel-Nr. | p.o. (mg/kg) | i.v (mg/kg) |
|---|---|---|
| erfindungsgemäße Verbindung (I) | > 30 | > 10 |

Die Tabelle zeigt, daß im Vergleich mit Modell II der Abstand der therapeutischen Wirkung und Blutdruckwirkung (i.v.) mindestens 100 beträgt.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

2,6-Dimethyl-1-n-propyl-4-(4-trifluormethylphenyl)-1,4-dihydropyridin-3,5-dicarbonsäuredimethylester

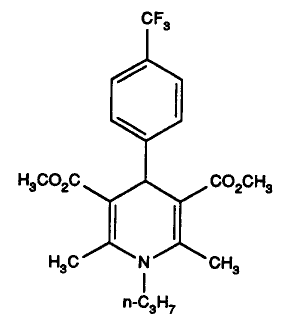

### (Verfahren D):

1) 2,95 g (0,008 mol) 2,6-Dimethyl-4-(4-trifluormethylphenyl)-1,4-dihydropyridin-3,5-dicarbonsäure-dimethylester werden in 25 ml 1,2-Dimethoxyethan gelöst, mit 0,30 g (0,01 mol) 80%igem Natriumhydrid und nach 30 min mit 1,70 g (0,01 mol) n-Propyliodid versetzt. Man rührt 3 Stunden bei Raumtemperatur nach, neutralisiert mit verdünnter Salzsäure und dampft im Vakuum ein. Der Rückstand wird durch Chromatographie über Kieselgel (Methylenchlorid) gereinigt.
   Ausbeute: 1,02 g (31,0% der Theorie).
   Schmelzpunkt: 102 - 104°C.

### (Verfahren D):

2) Unter Argon werden 0,3 g (0,01 mol) 80%iges Natriumhydrid in 50 ml Dimethylformamid (p.a.) bei 0°C mit 1,84 g (0,005 mol) 2,6-Dimethyl-4-(4-trifluormethylphenyl)-1,4-dihydropyridin-3,5-dicarbonsäuredimethylester versetzt. Anschließend gibt man langsam eine Lösung von 1,92 g (0,01 mol) Trifluormethansulfonsäure-n-propylester in 30 ml Methylenchlorid hinzu (hergestellt aus Trifluormethansulfonsäureanhydrid und n-Propanol in Methylenchlorid in Gegenwart von äquivalenten Mengen Pyridin) und rührt bei 0°C 1 h nach. Nach dem Versetzen mit Wasser wird mit Methylenchlorid extrahiert, über Natriumsulfat getrocknet und eingedampft. Der Rückstand wird durch Chromatographie über Kieselgel mit Methylenchiorid gereinigt. Man erhält 1,16g (56,6% der Theorie).
   Schmp.: 100-103°C

### (Verfahren B):

3) Eine Mischung aus 5,22 g (0,03 mol) 4-Trifluormethylbenzaldehyd, 7,04 g (0,06 mol) Acetessigsäuremethylester und 2,87 g (0,03 mol) n-Propylamin-Hydrochlorid [oder 1,78 g (0,03 mol) n-Propylamin und 3,47 g (0,03 mol) Pyridin-Hydrochlorid] in 20 ml Pyridin wird 20 Stunden unter Rückfluß gerührt. Nach dem Abdestillieren des Pyridins wird zwischen Wasser und Methylenchlorid verteilt, die organische Phase gewaschen mit Wasser, getrocknet über Natriumsulfat und eingedampft. Der Rückstand wird aus Methanol umkristallisiert.
   Schmp.: 102 - 104°C
   Ausbeute: 3,1 g (25,1% der Theorie)

### Verfahren C:

4) Unter Stickstoffschutz fügt man zu 20 ml Toluol 0,55 ml (5 mmol) Titantetrachlorid, anschließend 1 ml (10 mmol) Piperidin und rührt 5 min. Nach dem Zutropfen von 3,14 g (20 mmol) 3-n-Propylaminocrotonsäuremethylester fügt man 1,36 ml (10 mmol) 4-Trifluormethylphenylbenzaldehyd zu und rührt 3 Stunden bei Raumtemperatur. Zur Aufarbeitung wird 100 ml 5%ige Salzsäure zugesetzt und die organische Phase mit Essigester aufgenommen, die Essigesterlösung nacheinander mit 5% Salzsäure und mit Natriumbicarbonatlösung gewaschen. Nach dem Trocknen der Essigesterlösung über Natriumsulfat, Eindampfen und Verrühren des Rückstandes in n-Heptan erhält man 1,59g (38,7% der Theorie)
   Schmp.: 100-102°C

### Verfahren A:

5) Zu einer Lösung von 3,9 g (0,03 mol) Acetessigsäuremethylester und 8,16 g (0,03 mol) 2-(4-Trifluormethylbenzyliden)-acetessigsäuremethylester in 50 ml Pyridin fügt man 3,72g (0,039 mol) n-Propylamin-Hydrochlorid und erhitzt 5 Stunden unter Rückfluß. Man engt das Reaktionprodukt im Vakuum ein, nimmt den Rückstand in Methylenchlorid und Wasser auf, trennt die wäßrige Phase ab, trocknet die Methylenchloridlösung über Natriumsulfat und dampft ein. Der Rückstand wird durch Chromatographie über Kieselgel mit Methylenchlorid als Lösemittel gereinigt. Nach dem Umlösen aus n-Heptan erhält man 1,93 g (15,6% der Theorie).
   Schmp.: 102 - 104°C

## Patentansprüche

1. Die Verbindung 2,6-Dimethyl-1-n-propyl-4-(4-trifluormethylphenyl)-1,4-dihydropyridin-3,5-dicarbonsäuredimethylester der Formel (I)

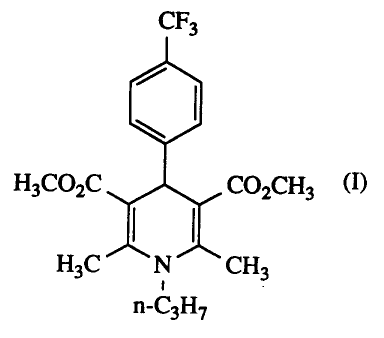

2. Die Verbindung gemäß Anspruch 1 zur Verwendung bei der Bekämpfung von akut und chronisch ischämischen Erkrankungen.

3. Verfahren zur Herstellung der Verbindung 2,6-Dimethyl-1-n-propyl-4-(4-trifluormethylphenyl)-1,4-dihydropyridin-3,5-dicarbonsäuredimethylester der Formel (I) 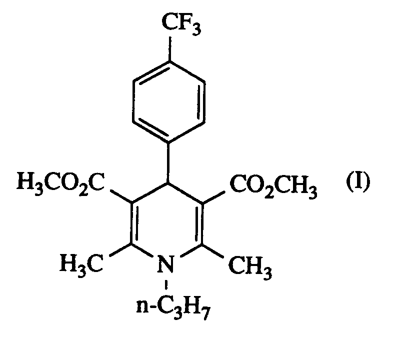 dadurch gekennzeichnet, daß man
[A] 2-(4-Trifluormethylbenzyliden)acetessigsäuremethylester der Formel (II) 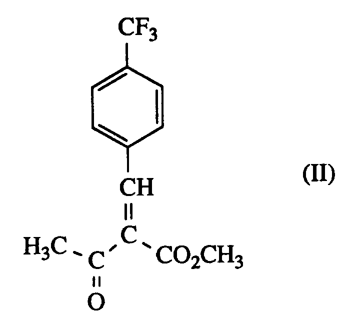 entweder direkt mit 3-n-Propylamino-crotonsäuremethylester, oder
mit Acetessigsäuremethylester und n-Propylamin-Hydrochlorid
in inerten Lösemitteln, gegebenenfalls in Anwesenheit einer Base / Säure umsetzt,
oder
[B] 4-Trifluormethylbenzaldehyd der Formel (III) 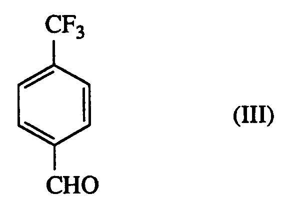 entweder mit Acetessigsäuremethylester und n-Propylaminhydrochlorid bzw. n-Propylamin und Pyridin-Hydrochlorid in Pyridin umsetzt,
oder
[C] zunächst unter Schutzgasatmosphäre Lewissäuren, vorzugsweise Titantetrachlorid, mit einer Base, vorzugsweise Piperidin, mit 3-n-Propylaminocrotonsäuremethylester in inerten Lösemitteln versetzt und anschließend 4-Trifluormethylbenzaldehyd der Formel (III) zufügt, oder
[D] 2,6-Dimethyl-4-(4-trifluormethylphenyl)-1,4-dihydropyridin-3,5-dicarbonsäuredimethylester der Formel (IV) 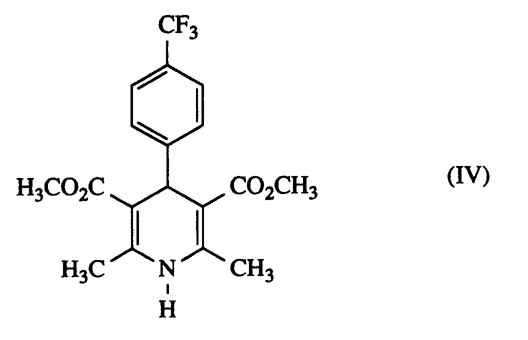 mit Alkylierungsmitteln, vorzugsweise n-Propyliodid oder Trifluormethansulfonsäure-n-propylester in inerten Lösemitteln und in Anwesenheit einer Base umsetzt.

4. Arzneimittel enthaltend die Verbindung gemäß Anspruch 1.

5. Verfahren zur Herstellung von Arzeimitteln, dadurch gekennzeichnet,daß man die Verbindung gemäß Anspruch 1 gegebenenfalls mit üblichen Hilfs- und Trägerstoffen in eine geeignete Applikationsform überführt.

6. Verwendung der Verbindung gemäß Anspruch 1 bei der Bekämpfung von ischämischen Erkrankungen, die mit Mikrozirkulationsstörungen verbunden sind.
